# EUROPEAN PATENT SPECIFICATION

(11) **EP 3 490 651 B1**
(45) Date of publication and mention of the grant of the patent: **01.09.2021**
(21) Application number: 17835018.7
(22) Date of filing: 21.07.2017
(51) Int. Cl.: A61M 16/08, A61M 16/00

(54) **DEVICE COMPRISING A FLUIDIC DIVERTER FOR ASSISTING BREATHING**
VORRICHTUNG MIT EINEM FLUIDABWEICHER ZUR UNTERSTÜTZUNG DER ATMUNG
DISPOSITIF COMPRENANT UN DÉFLECTEUR FLUIDIQUE POUR AIDER LA RESPIRATION

(30) Priority: 26.07.2016 US 201662366697 P
(43) Date of publication of application: 05.06.2019
(73) Proprietor: University of Cincinnati, Cincinnati, OH 45206 (US)
(72) Inventor: GUTMARK, Ephraim, Cincinnati, OH 45206 (US); OREN, Liran, Cincinnati, OH 45206 (US); KHOSLA, Sid, Cincinnati, OH 45206 (US)
(74) Representative: Moore, Michael Richard
(86) International application number: PCT/US2017/043257
(87) International publication number: WO 2018/022448

(56) References cited:
- WO-A1-2008/122045
- WO-A1-2015/020538
- US-A1- 2002 059 935
- US-A1- 2010 252 037
- US-A1- 2010 252 044
- US-A1- 2011 108 033
- US-A1- 2011 125 052
- US-A1- 2013 186 399
- US-A1- 2015 075 529

## Description

### RELATED APPLICATION

This application claims the benefit of United States Provisional Application Serial No. 62/366,697, filed July 26, 2016.

### FIELD OF THE INVENTION

The presently disclosed subject matter relates to devices and methods for assisting breathing in a subject. Specifically, the presently disclosed subject matter relates to an assisted breathing device comprising a fluidic diverter, which converts a pressurized airflow into trains of vortices that prevent upper airway obstruction in a subject without the need for a tight-fitting mask, and methods of using the same.

### BACKGROUND

An estimated 18-30 million Americans have obstructive sleep apnea (OSA), a chronic sleep-related breathing disorder that involves a decrease or complete halt in airflow despite an ongoing effort to breath. In OSA, muscles relax during sleep, causing soft tissues surrounding the pharynx to collapse and obstruct the upper airway. Airway obstruction leads to reduction or complete pauses in breathing during sleep. The brain responds to the resulting hypoxia and hypercapnia by alerting the body, causing a brief arousal from sleep that restores normal breathing. This disruptive cycle can occur hundreds of times each night.

Adverse outcomes independently associated with OSA include daytime sleepiness and fatigue, drowsy driving with increased risk of motor vehicle accidents, cardiovascular morbidities (e.g. hypertension, stroke, heart attack), metabolic syndrome and type 2 diabetes, nonalcoholic fatty liver disease, perioperative complications, and premature death. OSA is thought to account for 15-20% of the 40-50,000 deaths and almost 4,000,000 emergency department visits annually.

OSA is most likely to occur during the inspiration phase of respiration during sleep. Inspiration is associated with generation of negative intrathoracic pressure. Air enters into the airway because the pressure in the lungs is less than the atmospheric pressure outside the nares. For a patient with OSA, the collapse of the airway's soft tissue is likely to occur because negative pressure exists within the upper airway during inspiration.

Continuous Positive Air Pressure, or CPAP, is an effective and widely recognized therapy for obstructive sleep apnea, but is often under prescribed by physicians and under-utilized by patients. With CPAP therapy, the airflow is actively being "pushed" into the patient's airway. The pneumatic splint of CPAP maintains an open upper airway, thus preventing collapse of the soft tissues and airway obstruction. In the context of CPAP therapy, the term "positive airway pressure" means that the pressure from the airflow applied to the lumen of the airway is greater than the pressure applied to the airway from the surrounding soft tissue.

Currently marketed CPAP devices require a tightly sealed interface between the mask and patient's face to deliver pressurized air to the patient's upper airway, thereby creating positive pressure. In its basic form, the mask is placed over the patient's mouth and nose (i.e., a full face mask). If the mask is not sealed correctly, the air will then follow the path of least resistance, escaping out into the atmosphere where the pressure is lower. The patient will experience this as air bleeding out from the system, but more importantly, the positive pressure that is the basic of operation for CPAP therapy cannot be maintained. Consequently, the soft tissues surrounding the pharynx are more likely to collapse and obstruct the upper airway.

An alternative to a full face mask is a nasal apparatus that only works if the patient does not open his or her mouth at night. If the patient opens his or her mouth, the fully pressurized respiratory tract will lose pressurization as the air will follow the path of least resistance and rush out of the mouth. For patients that open their mouths, an additional apparatus to keep the mouth closed must be employed. Chin straps and bite guards are two approaches to help mouth breathers keep their mouths closed during sleep.

CPAP is efficacious when used nightly as prescribed, but is not well tolerated by patients. Studies show that somewhere between 46% and 83% of patients are not compliant with CPAP therapy and remove the CPAP device early in the night or skip use altogether. Compliance is poor due to the discomfort and inconvenience associated with CPAP. Issues that result in patient non-compliance include mask discomfort, nasal dryness, congestion, difficulty adapting to the unrelenting positive air pressure across the mouth and nose, abdominal bloating due to air entering the digestive tract, and facial skin irritation due to the requirement of a tight seal between the mask and face.

Accordingly, a need exists for alternative treatments for obstructive sleep apnea.

WO2015/020538A1 discloses a device for assisting breathing comprising a compressor for providing a pressurized airflow, a fluidic diverter fluidly coupled to the compressor and an actuator with two outlets.

### SUMMARY OF THE INVENTION

The present inventors have now developed a device and method for providing positive airway pressure to a subject in need thereof, without the need for a seal between the device and the face of a subject, by using a fluidic diverter to generate pulsatile vortex airflow for delivery to the nasal passageway of the subject.

In one embodiment not claimed, a method for assisting breathing in a subject in need thereof is provided, the method comprising: passing a pressurized airflow through a fluidic diverter, wherein the fluidic diverter converts the pressurized airflow into pulses of pressurized air that are diverted alternately to provide a first and a second train of pulses of pressurized air; and directing the first and second trains of pulses of pressurized air into a nasal passageway of a subject to assist breathing.

In another embodiment, a device for assisting breathing is provided, the device comprising: a compressor for providing a pressurized airflow; a fluidic diverter fluidly coupled to the compressor, the fluidic diverter comprising: an actuator comprising an inlet for receiving the pressurized airflow, a first outlet, and a second outlet; a flow path disposed on the actuator and fluidly coupled to the inlet and each of the first and second outlets; and a cover disposed on the actuator to enclose the flow path; wherein: the fluidic diverter converts the pressurized airflow into pulses of pressurized air diverted alternately into each of the first and second outlets; wherein the pulses of pressurized air exit the first and second outlets and enter a nasal passageway of a subject to assist breathing.

These and additional features provided by the embodiments described herein will be more fully understood in view of the following detailed description, in conjunction with the drawings.

### BRIEF DESCRIPTION OF THE DRAWINGS

The embodiments set forth in the drawings are illustrative and exemplary in nature and not intended to limit the subject matter defined by the claims. The following detailed description of the illustrative embodiments can be understood when read in conjunction with the following drawings, where like structure is indicated with like reference numerals and in which:
**FIGURE 1** depicts a top view of an exemplary fluidic diverter according to one or more embodiments described and illustrated herein.
**FIGURE 2** depicts an isometric view of an exemplary fluidic diverter according to one or more embodiments described and illustrated herein.
**FIGURE 3**(**A**) and **FIGURE 3**(**B**) together schematically depict an exemplary fluidic diverter according to one or more embodiments described and illustrated herein, wherein a pressurized airflow is diverted alternately between two outlets of the fluidic diverter.
**FIGURE 4** depicts an isometric view of an assisted breathing device comprising a fluidic diverter coupled to an airflow source according to one or more embodiments described and illustrated herein.
**FIGURE 5** depicts an assisted breathing device comprising a fluidic diverter coupled to an airflow source according to one or more embodiments described and illustrated herein.
**FIGURE 6** depicts an assisted breathing device comprising a fluidic diverter coupled to an airflow source, further comprising a nasal cannula and tubing for delivering a pulsatile vortex airflow to a subject, according to one or more embodiments described and illustrated herein.
**FIGURE 7** depicts an exploded perspective view of an assisted breathing device comprising a fluidic diverter, an airflow source, and a nasal cannula and tubing according to one or more embodiments described and illustrated herein.
**FIGURE 8** schematically depicts a nasal model replica and a fluidic diverter experiment arrangement.
**FIGURE 9** is a graph depicting the percent penetration of pulsating vortex airflow generated by a fluidic diverter versus continuous airflow in a nasal model.

### DETAILED DESCRIPTION OF THE INVENTION

The details of one or more embodiments of the presently-disclosed subject matter are set forth in this document. Modifications to embodiments described in this document, and other embodiments, will be evident to those of ordinary skill in the art after a study of the information provided in this document.

While the following terms are believed to be well understood by one of ordinary skill in the art, certain definitions are set forth to facilitate explanation of the presently-disclosed subject matter. Unless defined otherwise, all technical and scientific terms used herein have the same meaning as commonly understood by one of ordinary skill in the art to which the presently-disclosed subject matter belongs.

Unless otherwise indicated, all numbers expressing quantities of ingredients, properties such as reaction conditions, and so forth used in the specification and claims are to be understood as being modified in all instances by the term "about." Accordingly, unless indicated to the contrary, the numerical parameters set forth in this specification and claims are approximations that can vary depending upon the desired properties sought to be obtained by the presently-disclosed subject matter.

As used herein, the term "about," when referring to a value or to an amount of mass, weight, time, volume, concentration or percentage is meant to encompass variations of in some embodiments ±20%, in some embodiments ±10%, in some embodiments ±5%, in some embodiments ±1%, in some embodiments ±0.5*%*, and in some embodiments ±0.1% from the specified amount, as such variations are appropriate to perform the disclosed method.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

The devices and methods disclosed herein leverage the properties of pulsatile vortex airflow to overcome limitations of current CPAP devices and provide a well-tolerated therapy for OSA. Unlike the continuous airflow of CPAP methods, characterized by the continuous motion of air particles along specific path lines, vortex airflow is pulsating and formed of trains of toroid vortices. As rotating regions along an axis line, these vortices give the vortex airflow its unique properties. Vortices of pressurized air travel relatively far, compared to continuous airflow, without losing structure. The disclosed devices and methods deliver vortex airflow to the upper respiratory tract of a patient, eliminating the need for a tight-fitting interface (e.g., mask). Unlike continuous airflow, when vortex airflow is directed toward the nasal passageway of a patient, the majority of the vortex airflow will enter or push into the airway due to the celerity and penetration properties of the vortices. Once entered, the vortex airflow acts in the same manner as the continuous airflow of CPAP technology by applying the required pressure on the lumen to splint the airway.

The devices and methods disclosed herein employ a fluidic diverter flow control mechanism to convert continuous pressurized airflow into vortex airflow. Advantageously, fluidic diverters require a source of pressurized air, have no moving parts, add little weight to a device, and require no external power to operate (other than power required to operate the source of pressurized air). Further, fluidic diverters can be scaled to vary size, periodic frequency, and flow rate.

Generally, embodiments of the present disclosure produce trains of vortices, which operate to apply positive pressure to an airway independent of the surrounding conditions (i.e., whether a mask is sealed to the face or not). The laws of physics demonstrate that a pressurized airstream (gas) flows from an area of high pressure to an area of lower pressure. In the case of CPAP, the airstream takes the path of least resistance, such as out the side of an ill-fitting mask or out of a patient's mouth. However, embodiments of the present disclosure produce vortex airflow comprising trains of vortices with mass flow that is sufficient to produce enough pressure to keep the airways of the respiratory tract from collapsing. In addition, the trains of pulses (i.e., vortices) of pressurized air produced by the devices and methods described herein are able to penetrate the nasal passageway and provide positive airway pressure despite not being a closed system because of the match between the impedance of the trains of pulses of pressurized air and the impedance of the airway tract. Matching the impedance of the vortex airflow with the impedance of the airway tract is not a necessity for the devices and methods disclosed herein; however, doing so maximizes the vortex airflow entering into the nasal passages, thereby providing the most efficient therapy. The vortex airflow need only be directed toward the nares of the subject to give a desired effect of preventing respiratory occlusion. A nasal cannula positioned proximate to the nares of the subject will deliver the trains of vortices that selectively enter the respiratory tract without a loss of pressurized airflow from the mouth.

Various embodiments of devices comprising fluidic diverters and methods for assisting breathing using trains of pulses of pressurized air are described in detail below.

As used herein, the terms "pulsatile airflow," "pulsatile vortex airflow," "vortex airflow," and "pulses of pressurized air" are used interchangeably to refer to a pulsed airflow, wherein each pulse comprises a toroid vortex of pressurized air. A series of such pulses forms a train of pulses or vortices of pressurized air.

Fluidic diverters are well known in the art of aerodynamics for use in controlling fluid flow; however, the application of fluidic diverters to avert obstruction of the upper airway and assist breathing, particularly in the treatment of obstructive sleep apnea, represents a significant advancement over current CPAP technologies. The skilled artisan will appreciate that various configurations and designs of fluidic diverters may be employed in the devices and methods described herein.

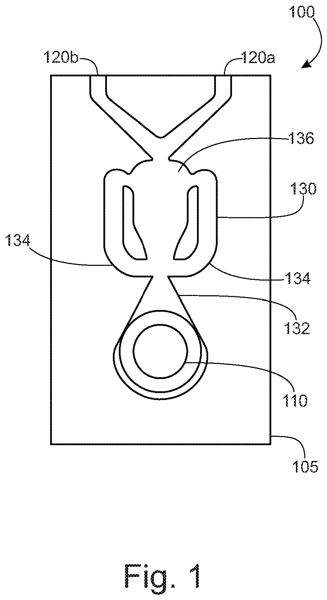

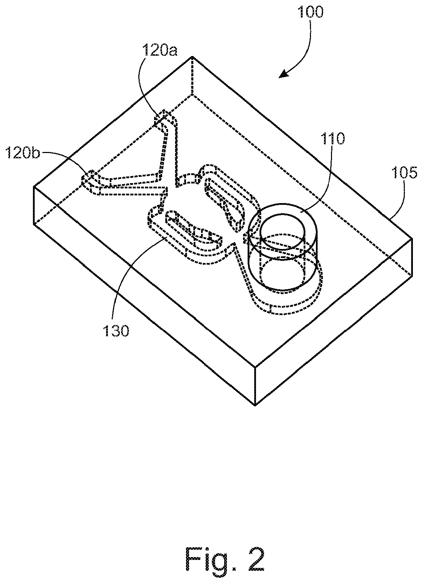

Referring now to Figs. 1-2, an exemplary fluidic diverter **100** for assisting breathing is depicted. The fluidic diverter **100** generally includes an actuator **105** comprising an inlet **110** for receiving a pressurized airflow, a first outlet **120a** and a second outlet **120b** for directing trains of pulses of pressurized air to a subject, and a flowpath **130** disposed on the actuator **105.** The flowpath **130** fluidly couples the inlet **110** and each of the outlets **120a** and **120b.** In one embodiment, a cover (not shown) is disposed on the actuator **105** to enclose the flowpath **130.** In another embodiment, the actuator **105** and the cover are engineered as a unitary structure, whereby the flowpath **130** is enclosed internally.

The flowpath **130** may comprise a variety of configurations suitable for generating a pulsatile airflow. In the exemplary fluidic diverter **100** of Fig. 1, the flowpath comprises a converging region **132,** a feedback channel **134,** and a chamber **136.**

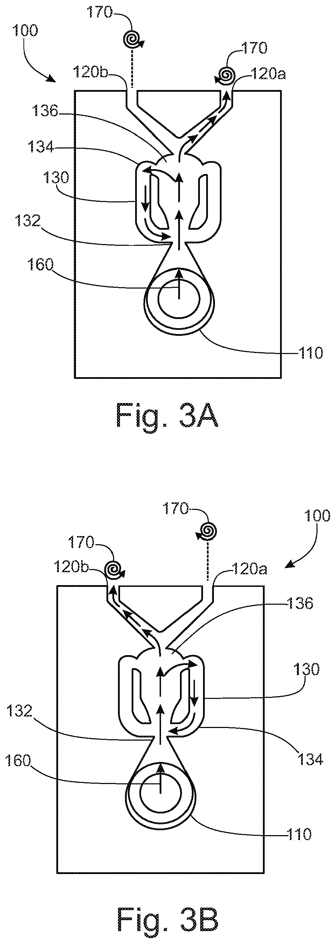

Referring to Fig. 3(A), as pressurized airflow **160** enters the fluidic diverter via the inlet **110** and passes through the converging region **132,** the airflow **160** becomes bistable in nature and steers to a first outlet **120a** due to the Coanda effect, or the tendency of a fluid jet to stay attached to an adjacent curved surface. A portion of the airflow **160** is transmitted back through the feedback channel **134.** Movement of a portion of the airflow **160** through the feedback channel **134** switches the jet attachment from the first outlet **120a** to the second outlet **120b,** as shown in Fig. 3(B). The process then repeats as a portion of the airflow **160** moves through the feedback channel **134,** switching the jet attachment back to the first outlet **120a.** In this way, the pressurized airflow **160** is diverted alternately into the two outlets **120a** and **120b** of the actuator **105.** The fluidic diverter **100** converts the continuous pressurized airflow **160** into two trains of pulses (i.e., vortices) **170** of pressurized air, which exit the fluidic diverter **100** via the outlets **120a** and **120b, respectively.**

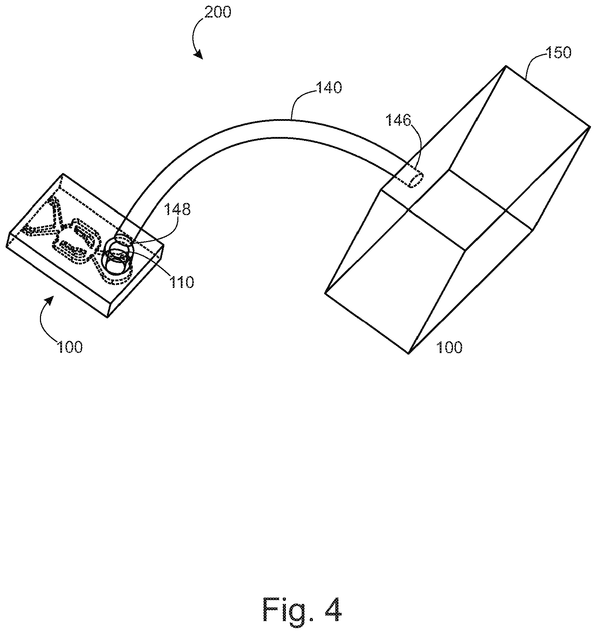

Referring to Figs. 4-7, embodiments of an assisted breathing device **200** are represented. The fluidic diverter **100** is fluidly coupled to a pressurized airflow source **150** via a tube **140,** having a first end **146** coupled to the pressurized airflow source **150** and a second end **148** coupled to inlet **110** of the fluidic diverter **100.** The skilled artisan will appreciate that various pressurized airflow sources are suitable for use in the devices and methods described herein. For example, in certain embodiments, the pressurized airflow source **150** is a compressor, a pump, a piston, a syringe, a bellows, or other suitable source known in the art. In a specific embodiment, the pressurized airflow source **150** is compressor, such as a standard CPAP machine.

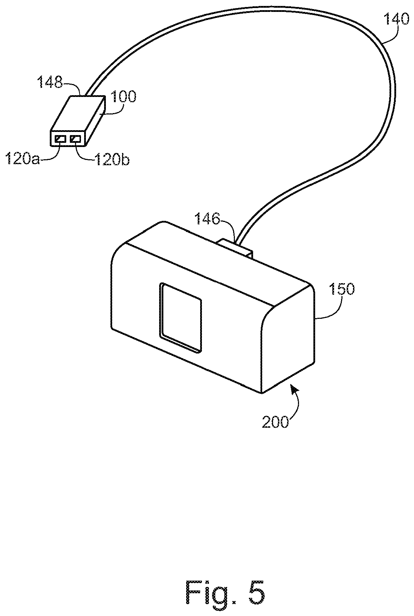

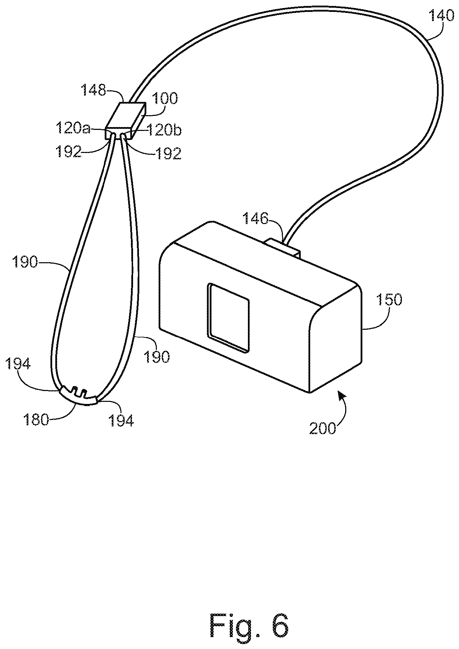

Figs. 5 and 6 depict embodiments of an assisted breathing device **200** wherein the pressurized airflow source **150** is fluidly coupled to the fluidic diverter **100** via a tube **140,** having a first end **146** coupled to the pressurized airflow source **150** and a second end **148** coupled to the inlet (not shown) of the fluidic diverter **100.** The fluidic diverter **100** converts the pressurized airflow into two trains of pulses of pressurized air, which exit the fluidic diverter **100** via the outlets **120a** and **120b,** respectively. In Fig. 5, an embodiment is depicted wherein the fluidic diverter **100** is configured such that the outlets **120a** and **120b** are positioned proximate to a subject's nostrils to direct the trains of pulses of pressurized air into the nasal passageway of the subject without the need for a seal between the outlets **120a** and **120b** and the nasal passageway of the subject. In such an embodiment, a nasal cannula or mask is not required to apply positive airway pressure to the subject's upper respiratory tract.

Fig. 6 depicts an embodiment of an assisted breathing device **200,** wherein the outlets **120a** and **120b** are fluidly coupled to a nasal cannula **180** via first and second tubes **190.** Each of the first and second tubes **190** has a first end **192** coupled to the outlet **120a** or **120b,** respectively, and a second end **194** coupled to the nasal cannula **180.** Unlike traditional CPAP technology, the device **200** functions to avert respiratory tract occlusion even when a seal is not present between the nasal cannula **180** and the subject's nostrils.

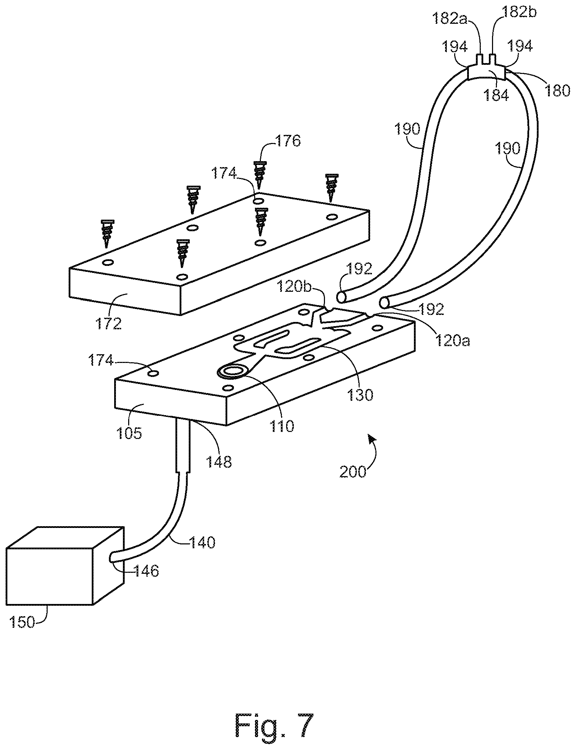

Fig. 7 shows an exploded view of an embodiment of an assisted breathing device **200.** A pressurized airflow source **150** is fluidly coupled to the actuator **105** via a tube **140** having a first end **146** fluidly coupled to the pressurized airflow source **150** and a second end **148** fluidly coupled to the inlet **110.** The actuator comprises outlets **120a** and **120b** for discharging trains of pulses of pressurized air. The outlets **120a** and **120b** and the inlet **110** are fluidly coupled via the flowpath **130.** A cover 172 is disposed on the actuator **105** to enclose the flowpath **130.** In one embodiment, the cover **172** and the actuator **105** are fastened together to enclose the flowpath **130.** The skilled artisan will appreciate that various fasteners are suitable for securing the actuator **105** and the cover **172,** including adhesive, welding, clamps, nails, screws, and the like. Fig. 7 depicts an exemplary embodiment, wherein the actuator **105** and the cover **172** are secured via a plurality of screws **176** and threaded holes **174** for receiving the screws **176.** First and second tubes **190** have a first end **192** that fluidly couples to the outlet **120a** or **120b** and a second end **194** that fluidly couples to the nasal cannula **180.**

In one embodiment, the nasal cannula **180** is comprised of a chamber **184** fluidly coupled to a first prong **182a** and a second prong **182b,** wherein each of said prongs **182a** and **182b** is configured to be positioned proximate to a nostril of a subject. The chamber **184** is fluidly coupled to each of the first and second tubes **190.** In a specific embodiment, the nasal cannula **180** is configured to keep the two trains of vortices separated as they enter the nostrils of a subject. In one embodiment, the nasal cannula **180** comprises a septum (not shown) for dividing the chamber **184** into a first compartment for receiving pulsatile airflow from the first tube **190** and a second compartment for receiving pulsatile airflow from the second tube **190.** In such an embodiment, the first compartment of the cannula **180** is fluidly coupled to the first prong **182a** and the second compartment is fluidly coupled to the second prong **182b.** The nasal cannula **180** having a septum maintains two distinct trains of vortices, each entering a separate nostril of the user.

As stated above, the fluidic diverter **100** creates two streams of vortex airflow, i.e., two trains of pulses of pressurized air. Conventional jets of air or other fluid, such as those used by existing ventilators and CPAP devices, produce a laminar flow of air that transitions into turbulent flow of air, which disperses very quickly as a free jet. However, the vortex airflow generated by embodiments of the present disclosure are defined by trains of vortices **170** that remain coherent in shape (i.e., the trains of vortices have a lower rate of dispersion) and produce high rates of the flow. The fluidic diverter **100** generates trains of vortices **170** as the airflow is diverted between the two outlets **120** of the fluidic diverter. Each vortex **170** then advects downstream under its own induced velocity. Based on the operating frequency of the fluidic diverter **100,** this process can be repeated tens/hundreds/thousands of times per second. Patients may not be able to discern that the airflow is actually composed from a train of vortices **170,** and may experience the trains of vortices **170** as if they were a continuous jet.

The enhanced celerity of the trains of vortices **170** is associated with the ability of the flow to sustain high momentum (i.e., penetration of the flow) for longer distances. The vortices **170** that exit the outlets **120** or the nasal cannula **180** will enter the nostril and respiratory tract without significant reflection and/or loss to the environment. The pressurized airflow source **150** and the fluidic diverter **100** generate pressure (i.e., "power") that is sufficient to keep the passageways of the respiratory tract from collapsing and causing occlusion.

In addition to higher penetration of flow, another advantage of the embodiments described herein is the ability to direct air pressures specifically to the occluded area of the respiratory tract. Use of trains of vortices **170** enables airstreams to be produced that will selectively flow to areas of greater resistance based on the principle of impedance matching. The coherent structure of the flow, together with the ability to match the impedance of the trains of vortices **170** with the impedance of the airway (e.g., nasal passageways/respiratory tract), eliminates the need for a closed system that requires a mask. The impedance of the trains of pulses of pressurized air **170** may be matched to the impedance of the patient's airway (i.e., nasal passageways/respiratory tract).

According to the concept of impedance matching, the maximum energy transfer (i.e., with minimum reflections) occurs when the impedance of the source (input) matches the impedance of the load (output). Impedance is a general term used to describe resistance. While often associated with electric circuits, the concept of impedance is also applicable to the behavior of flow in fluid mechanics. The impedance of the flow in a system (i.e., tube, channel, nasal cavities, etc.) is determined from the ratio of pressure to the flow rate. Like an electrical circuit, flow impedance is a function of frequency. Thus, impedance can be modified for pulsatile airflow. In the context of electrical circuits, impedance matching is a method used for maximizing power (energy) transfer of an electrical wave between a load and a source. Analogized to fluid flow, impedance matching is a method used for maximizing the transfer of waves from one medium to the next with minimum loss to reflections. The impedance value of a nasal airway is patient-specific, and determined by the ratio of lung pressure and flow rate (i.e., velocity of the flow moving in the airway). When the impedance values are matched between a nasal airway and a vortex airflow, maximum flow is transferred from outside the nares into the nasal passageway, with minimal loss to reflections.

As described above, the trains of vortices **170** are formed by passing a pressurized airflow **160** through the fluidic diverter. A frequency of the trains of vortices can be varied by adjusting factors such as the geometry of the actuator **105,** the flowpath **130,** and the input pressure of the airflow **160** from the airflow source **150.** The impedance of the pressurized airflow **160** is directly related to the frequency of pulses and, thus, the fluidic diverter **100** described herein may produce trains of vortices **170** with varying impedance. In certain embodiments, the frequency of pulses of pressurized air ranges from about 1 Hz to about 50 kHz.

Advantageously, a magnitude of a vortex of pressurized air is adjusted in the devices and methods disclosed herein by adjusting a magnitude of the airflow entering the fluidic diverter **100.**

Generally, different obstructive sleep apnea patients require different levels of air pressure to open their airways of the respiratory tract. This variation in the required air pressure necessary to open subjects' airways may be caused by variation in the locations and severity of subjects' airway blockage and/or degree of occlusion.

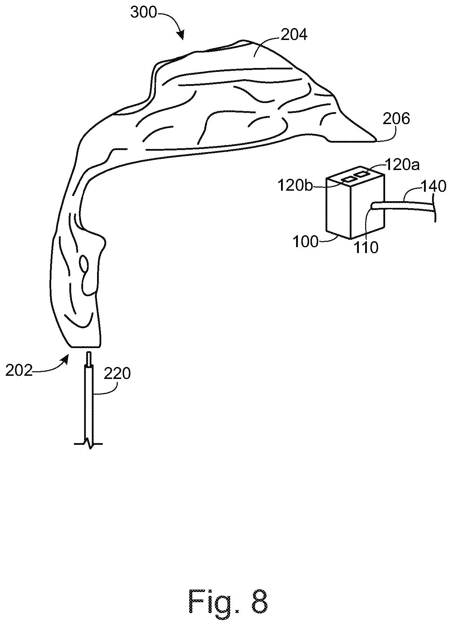

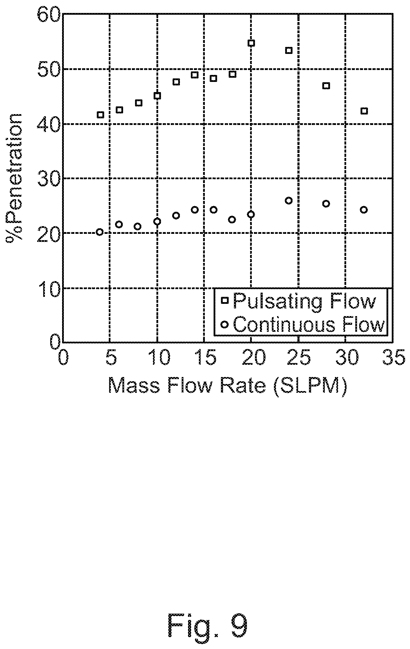

Referring to Fig. 8, a device for assisted breathing **200** according to Figs. 1-7 was built for evaluation purposes. Additionally, a 2:1 realistic nasal model **300** replica of the airway passage **204** from the nostrils **206** to the epiglottis **202** was constructed from CT scans taken along the upper nasal airway. A fluidic diverter **100** was placed at an approximate 10 mm standoff distance from the nostrils **206** of the nasal model **300.** Its penetration (i.e., efficiency) was assessed by comparing the input and output (volume) flow rates with the aid of a probe **220.** If airflow in the experiment were delivered as in current CPAP therapy (i.e., via a tight fitting mask), the input and output flow rates would have been equal because no flow would have leaked to the ambient. As shown in Fig. 9, results indicate that when applying the vortex airflow at a small offset from the nares **206,** more than 50% of input flow rate penetrated (i.e., was measured at the exit **202** of the model). The experiment also compared delivering continuous airflow using the same input conditions (i.e., applying airflow approximately 10 mm from the nares). Results showed that no more than 25% of the input flow rate penetrated into the model. Hence, in the absence of a mask, the majority of continuous airflow was lost to the ambient, while the vortex airflow was more than twice as likely to enter the airway model (compared with the continuous airflow).

In one embodiment, a method for assisting breathing in a subject in need thereof is provided, the method comprising passing a pressurized airflow through a fluidic diverter, wherein the fluidic diverter converts the pressurized airflow into pulses of pressurized air that are diverted alternately to provide a first train and a second train of pulses of pressurized air; and directing the first and second trains of pulses of pressurized air into a nasal passageway of a subject to assist breathing. In certain embodiments, the first and second trains of pulses of pressurized air enter the nasal passageway and avert respiratory tract occlusion, thereby treating obstructive sleep apnea. In a specific embodiment, each of the pulses of pressurized air comprises a toroid vortex of pressurized air.

In a specific embodiment, the method further comprises generating the pressurized airflow by a compressor. In another embodiment, a magnitude of the vortex of pressurized air is adjusted by adjusting a magnitude of the airflow entering the fluidic diverter.

In one embodiment, the first train of pulses of pressurized air exits the fluidic diverter via a first outlet, and the second train of pulses of pressurized air exits the fluidic diverter via a second outlet.

In an embodiment, the first and second trains of pulses of pressurized air are directed into the nasal passageway of the subject by positioning the first outlet proximate to a first nostril of a subject and the second outlet proximate to a second nostril of the subject, without the use of a nasal cannula.

Alternatively, in another embodiment, the first and second trains of pulses of pressurized air are directed into the nasal passageway of the subject through a nasal cannula fluidly coupled to each of the first and second outlets of the fluidic diverter, wherein the nasal cannula is configured to be positioned proximate to the nostrils of the subject. In such an embodiment, a seal is not present or necessary between the nasal cannula and the nostrils of the subject in order to prevent airway occlusion.

In one embodiment, the nasal cannula is fluidly coupled to the first outlet via a first tube and is fluidly coupled to the second outlet via a second tube, wherein each of said first and second tubes delivers a train of pulses of pressurized air to the nasal passageway of the subject via the nasal cannula.

In a specific embodiment, the methods described herein further comprise matching a frequency of the pulses of pressurized air to an impedance of the nasal passageway. In a very specific embodiment, the frequency of the pulses of pressurized air ranges from about 1 Hz to about 50 kHz.

In another embodiment, a device for assisting breathing is provided, comprising: a compressor for providing a pressurized airflow; a fluidic diverter fluidly coupled to the compressor, the fluidic diverter comprising: an actuator comprising an inlet for receiving the pressurized airflow, a first outlet, and a second outlet; a flow path disposed on the actuator and fluidly coupled to the inlet and each of the first and second outlets; and a cover disposed on the actuator to enclose the flow path; wherein: the fluidic diverter converts the pressurized airflow into pulses of pressurized air diverted alternately into each of the first and second outlets; wherein the pulses of pressurized air exit the first and second outlets and enter a nasal passageway of a subject to assist breathing. In certain embodiments, each of said pulses of pressurized air comprises a toroid vortex of pressurized air. Advantageously, the devices disclosed herein do not require a seal between the first and second outlets and the nostrils and/or nasal passageway of the subject.

In certain embodiments, the device further comprises a first tube having a first end and a second end, the first end coupled to the first outlet and the second end coupled to a nasal cannula; and a second tube having a first end and a second end, the first end coupled to the second outlet and the second end coupled to the nasal cannula, wherein the nasal cannula is configured to be positioned proximate to nostrils of a subject. Advantageously, the devices disclosed herein do not require a seal between the nasal cannula and the nostrils and/or nasal passageway of the subject.

In a more specific embodiment, the nasal cannula comprises a chamber fluidly coupled to a first prong and a second prong, wherein each of said prongs is configured to be positioned proximate to a nostril of a subject. Optionally, the nasal cannula comprises a septum for dividing the chamber into a first compartment for receiving pulses of pressurized air from the first tube and a second compartment for receiving pulses of pressurized air from the second tube, wherein the first compartment is fluidly coupled to the first prong and wherein the second compartment is fluidly coupled to the second prong.

In one embodiment, a frequency of the pulses of pressurized air is such that an impedance of the pulses of pressurized air is matched to an impedance of the nasal passageway.

In another embodiment, the device assists breathing in a subject suffering from obstructive sleep apnea by providing positive airway pressure to avert respiratory tract occlusion.

While particular embodiments of the present invention have been illustrated and described, it would be obvious to one skilled in the art that various other changes and modifications can be made without departing from the scope of the invention. It is therefore intended to cover in the appended claims all such changes and modifications that are within the scope of this invention.

## Claims

1. A device for assisting breathing, comprising:
a compressor (150) for providing a pressurized airflow;
a fluidic diverter (100) fluidly coupled to the compressor, the fluidic diverter comprising:
an actuator (105) comprising an inlet for receiving the pressurized airflow, a first outlet (120a), and a second outlet (120b),
a flow path (130) disposed on the actuator and fluidly coupled to the inlet and each of the first and second outlets; and
a cover disposed on the actuator to enclose the flow path;
wherein:
the fluidic diverter converts the pressurized airflow into pulses of pressurized air diverted alternately into each of the first and second outlets; wherein the pulses of pressurized air exit the first and second outlets and enter a nasal passageway of a subject to assist breathing.

2. The device of claim 1, further comprising:
a first tube having a first end and a second end, the first end coupled to the first outlet and the second end coupled to a nasal cannula; and
a second tube having a first end and a second end, the first end coupled to the second outlet and the second end coupled to the nasal cannula,
wherein the nasal cannula is configured to be positioned proximate to nostrils of a subject.

3. The device of claim 2, wherein the nasal cannula comprises a chamber fluidly coupled to a first prong and a second prong, wherein each of said prongs is configured to be positioned proximate to a nostril of a subject.

4. The device of claim 3, wherein the nasal cannula comprises a septum for dividing the chamber into a first compartment for receiving pulses of pressurized air from the first tube and a second compartment for receiving pulses of pressurized air from the second tube, wherein the first compartment is fluidly coupled to the first prong and wherein the second compartment is fluidly coupled to the second prong.

5. The device of claim 1, wherein each of said pulses of pressurized air comprises a toroid vortex of pressurized air.

6. The device of claim 5, wherein a frequency of the pulses of pressurized air is such that an impedance of the pulses of pressurized air is matched to an impedance of the nasal passageway.

7. The device of claim 1, wherein the device assists breathing in a subject suffering from obstructive sleep apnea by providing positive airway pressure to avert respiratory tract occlusion.

8. The device of claim 1, wherein the device does not require a seal between the first and second outlets and the nasal passageway of the subject.

9. The device of claim 2, wherein the device does not require a seal between the nasal cannula and the nostrils of the subject.

10. The device of claim 6, wherein the frequency of the pulses of pressurized air is from about 1 Hz to about 50 kHz.

11. The device of claim 1, wherein the actuator and the cover are engineered as a unitary structure to enclose the flow path.

12. The device of claim 1, wherein the flow path comprises a converging region fluidly coupled to the inlet, a chamber disposed between the converging region and the first and second outlets, and a first and second feedback channel, each of said feedback channels fluidly coupled to the chamber and the converging region, wherein pressurized airflow passes through the converging region and is diverted alternately into each of the first and second outlets.

13. The device of claim 12, wherein transmission of a portion of the airflow alternately between the first and second feedback channels diverts the airflow alternately into each of the first and second outlets.

14. The device of claim 1, wherein the fluidic diverter is fluidly coupled to the compressor via a tube, wherein the compressor comprises a continuous positive air pressure (CPAP) machine.

15. The device of claim 1, wherein the pulses of pressurized air diverted alternately into each of the first and second outlets comprise trains of vortices of air that remain coherent in shape.

## Patentansprüche

1. Eine Vorrichtung zur Unterstützung der Atmung, die Folgendes beinhaltet:
einen Kompressor (150) zum Bereitstellen eines Druckluftstroms;
einen Fluidumleiter (100), der fluidisch mit dem Kompressor gekoppelt ist, wobei der Fluidumleiter Folgendes beinhaltet:
einen Aktuator (105), der einen Einlass zum Aufnehmen des Druckluftstroms, einen ersten Auslass (120a) und einen zweiten Auslass (120b) beinhaltet,
einen Strömungsweg (130), der auf dem Aktuator angeordnet ist und fluidisch mit dem Einlass und jedem von dem ersten und zweiten Auslass gekoppelt ist; und
eine Abdeckung, die auf dem Aktuator angeordnet ist, um den Strömungsweg zu umschließen;
wobei:
der Fluidumleiter den Druckluftstrom in Druckluftstöße umwandelt, die abwechselnd in jeden von dem ersten und zweiten Auslass umgeleitet werden; wobei die Druckluftstöße aus dem ersten und zweiten Auslass austreten und in eine Nasenpassage eines Individuums eintreten, um die Atmung zu unterstützen.

2. Vorrichtung gemäß Anspruch 1, die ferner Folgendes beinhaltet:
einen ersten Schlauch mit einem ersten Ende und einem zweiten Ende, wobei das erste Ende mit dem ersten Auslass gekoppelt ist und das zweite Ende mit einer Nasenkanüle gekoppelt ist; und
einen zweiten Schlauch mit einem ersten Ende und einem zweiten Ende, wobei das erste Ende mit dem zweiten Auslass gekoppelt ist und das zweite Ende mit der Nasenkanüle gekoppelt ist,
wobei die Nasenkanüle zum Positionieren in der Nähe der Nasenlöcher eines Individuums ausgelegt ist.

3. Vorrichtung gemäß Anspruch 2, wobei die Nasenkanüle eine Kammer beinhaltet, die fluidisch mit einem ersten Rohrund einem zweiten Rohr gekoppeltist, wobei jedes der Rohre zum Positionieren in der Nähe des Nasenlochs eines Individuums ausgelegt ist.

4. Vorrichtung gemäß Anspruch 3, wobei die Nasenkanüle eine Scheidewand zum Aufteilen der Kammer in ein erstes Abteil zum Aufnehmen von Druckluftstößen von dem ersten Schlauch und ein zweites Abteil zum Aufnehmen von Druckluftstößen von dem zweiten Schlauch beinhaltet, wobei das erste Abteil fluidisch mit dem ersten Rohr gekoppelt ist und wobei das zweite Abteil fluidisch mit dem zweiten Rohr gekoppelt ist.

5. Vorrichtung gemäß Anspruch 1, wobei jeder der Druckluftstößen einen ringförmigen Druckluftwirbel beinhaltet.

6. Vorrichtung gemäß Anspruch 5, wobei eine Frequenz der Druckluftstöße solcherart ist, dass eine Impedanz der Druckluftstöße einer Impedanz der Nasenpassage entspricht.

7. Vorrichtung gemäß Anspruch 1, wobei die Vorrichtung die Atmung bei einem an obstruktiver Schlafapnoe leidenden Individuum unterstützt, indem sie positiven Atemwegsdruck bereitstellt, um den Verschluss der Atemwege zu verhindern.

8. Vorrichtung gemäß Anspruch 1, wobei die Vorrichtung keine Abdichtung zwischen dem ersten und zweiten Auslass und der Nasenpassage des Individuums erfordert.

9. Vorrichtung gemäß Anspruch 2, wobei die Vorrichtung keine Abdichtung zwischen der Nasenkanüle und den Nasenlöchern des Individuums erfordert.

10. Vorrichtung gemäß Anspruch 6, wobei die Frequenz der Druckluftstöße von etwa 1 Hz bis etwa 50 kHz beträgt.

11. Vorrichtung gemäß Anspruch 1, wobei der Aktuator und die Abdeckung als einstückige Struktur hergestellt sind, um den Strömungsweg zu umschließen.

12. Vorrichtung gemäß Anspruch 1, wobei der Strömungsweg Folgendes beinhaltet: einen zusammenlaufenden Bereich, der fluidisch mit dem Einlass gekoppelt ist, eine Kammer, die zwischen dem zusammenlaufenden Bereich und dem ersten und zweiten Auslass angeordnet ist, und einen ersten und zweiten Rückkopplungskanal, wobei jeder von den Rückkopplungskanälen fluidisch mit der Kammer und dem zusammenlaufenden Bereich gekoppelt ist, wobei der Druckluftstrom durch den zusammenlaufenden Bereich hindurchläuft und abwechselnd in den ersten und zweiten Auslass umgeleitet wird.

13. Vorrichtung gemäß Anspruch 12, wobei die Übertragung eines Anteils des Luftstroms abwechselnd zwischen dem ersten und zweiten Rückkopplungskanal den Luftstrom abwechselnd in jeden von dem ersten und zweiten Auslass umleitet.

14. Vorrichtung gemäß Anspruch 1, wobei der Fluidumleiter über einen Schlauch fluidisch mit dem Kompressor gekoppelt ist, wobei der Kompressor eine CPAP-Maschine (kontinuierlicher positiver Atemwegdruck) beinhaltet.

15. Vorrichtung gemäß Anspruch 1, wobei die abwechselnd in jeden von dem ersten und zweiten Auslass umgeleiteten Druckluftstöße Luftwirbelfolgen, die in ihrer Gestalt zusammenhängend bleiben, beinhalten.

## Revendications

1. Dispositif pour aider la respiration, comprenant :
un compresseur (150) pour fournir un flux d'air sous pression ;
un déflecteur fluidique (100) couplé de manière fluide au compresseur, le déflecteur fluidique comprenant :
un actionneur (105) comprenant une entrée pour recevoir le flux d'air sous pression, une première sortie (120a) et une seconde sortie (120b),
une voie d'écoulement (130) placée sur l'actionneur et couplée de manière fluide à l'entrée et à chacune des première et seconde sorties ; et
un couvercle placé sur l'actionneur pour englober la voie d'écoulement;
dans lequel :
le déflecteur fluidique convertit le flux d'air sous pression en impulsions d'air sous pression déviées alternativement dans chacune des première et seconde sorties ; dans lequel les impulsions d'air sous pression sortent des première et seconde sortie s et entrent dans un passage nasal d'un sujet pour aider la respiration.

2. Dispositif selon la revendication 1, comprenant en outre :
un premier tube ayant une première extrémité et une seconde extrémité, la première extrémité étant couplée à la première sortie et la seconde extrémité étant couplée à une canule nasale ; et
un second tube ayant une première extrémité et une seconde extrémité, la première extrémité étant couplée à la seconde sortie et la seconde extrémité étant couplée à la canule nasale,
dans lequel la canule nasale est conçue pour être positionnée à proximité des narines d'un sujet.

3. Dispositif selon la revendication 2, dans lequel la canule nasale comprend une chambre couplée de manière fluide à une première broche et à une seconde broche, dans lequel chacune desdites broches est conçue pour être positionnée à proximité d'une narine d'un sujet.

4. Dispositif selon la revendication 3, dans lequel la canule nasale comprend un septum pour diviser la chambre dans un premier compartiment pour recevoir les impulsions d'air sous pression du premier tube et un second compartiment pour recevoir les impulsions d'air sous pression du second tube, dans lequel le premier compartiment est couplé de manière fluide à la première broche et dans lequel le second compartiment est couplé de manière fluide à la seconde broche.

5. Dispositif selon la revendication 1, dans lequel chacune desdites impulsions d'air sous pression comprend un vortex torique d'air sous pression.

6. Dispositif selon la revendication 5, dans lequel une fréquence des impulsions d'air sous pression est telle qu'une impédance des impulsions d'air sous pression correspond à une impédance du passage nasal.

7. Dispositif selon la revendication 1, dans lequel le dispositif aide la respiration chez un sujet atteint d'une apnée obstructive du sommeil en fournissant une pression positive dans les voies aériennes pour éviter une occlusion des voies respiratoires.

8. Dispositif selon la revendication 1, dans lequel le dispositif ne nécessite pas une interface étanche entre les première et seconde sorties et le passage nasal du sujet.

9. Dispositif selon la revendication 2, dans lequel le dispositif ne nécessite pas une interface étanche entre la canule nasale et les narines du sujet.

10. Dispositif selon la revendication 6, dans lequel la fréquence des impulsions d'air sous pression varie d'environ 1 Hz à environ 50 kHz.

11. Dispositif selon la revendication 1, dans lequel l'actionneur et le couvercle sont réalisés comme une structure unitaire pour englober la voie d'écoulement.

12. Dispositif selon la revendication 1, dans lequel la voie d'écoulement comprend une région convergente couplée de manière fluide à l'entrée, une chambre placée entre la région convergente et les première et seconde sorties, et un premier et un second canal de retour, chacun desdits canaux de retour étant couplé de manière fluide à la chambre et à la région convergente, dans lequel le flux d'air sous pression passe au travers la région convergente et est dévié alternativement dans chacune des première et seconde sorties.

13. Dispositif selon la revendication 12, dans lequel la transmission d'une partie du flux d'air alternativement entre les premier et second canaux de retour dévie le flux d'air alternativement dans chacune des première et seconde sorties.

14. Dispositif selon la revendication 1, dans lequel le déflecteur fluidique est couplé de manière fluide au compresseur par un tube, dans lequel le compresseur comprend une machine de pression d'air positive continue (CPAP).

15. Dispositif selon la revendication 1, dans lequel les impulsions d'air sous pression déviées alternativement dans chacune des première et seconde sorties comprennent des trains de vortex d'air dont la forme reste cohérente.
